# EUROPEAN PATENT SPECIFICATION

(11) **EP 0 627 235 B1**
(45) Date of publication and mention of the grant of the patent: **10.06.1998**
(21) Application number: 94105720.0
(22) Date of filing: 13.04.1994
(51) Int. Cl.: A61N 1/05, A61B 5/04

(54) **Medical system**
Medizinisches System
Système medical

(30) Priority: 01.06.1993 SE 9301855
(43) Date of publication of application: 07.12.1994
(73) Proprietor: Pacesetter AB, 171 95 Solna (SE)
(72) Inventor: Lindegren, Ulf, S-122 35 Enskede (SE); Guerola, Modesto, ES-08007 Barcelona (ES)
(74) Representative: Lettström, Richard Wilhelm

(56) References cited:
- US-A- 4 807 632
- US-A- 4 815 469
- US-A- 5 058 586

## Description

The invention relates to a medical system, intended to be connected to living tissue, comprising a medical device.

A plurality of different medical devices, such as pacemakers and defibrillators, are currently employed in systems for connection to living tissue for sensing tissue function or treating said tissue. The devices can be implantable or extracorporeal. The devices are connected to living tissue by metallic conductors. When a plurality of devices are utilized in a system connected to the living tissue, the incorporated devices can even be interconnected with metallic conductors so information can be exchanged by the devices.

Metallic leads can cause problems, especially in implanted systems. A pacemaker conductor could e.g. pick up a defibrillation pulse, resulting in spurious detection or even damage to pacemaker electronics. The metallic conductors are usually arranged in insulated sheaths made of a polymer, such as silicone, to keep them from having any effect on surrounding tissue which is not to be sensed or stimulated. This means that such leads cannot be made as thin as would be desirable.

One object of the present invention is to achieve a medical system, as noted above, in which the number of metallic conductors is minimized.

One such medical system is achieved in accordance with the invention in that the medical system further comprises at least one optical conductor with a proximal end and a distal end, connected to the medical device at the proximal end, a first conversion means, located at the proximal end of the optical conductor for converting optical signals into electrical signals and/or electrical signals into optical signals, a second conversion means, located at the distal end of the optical conductor, for converting optical signals into electrical signals and/or electrical signals into optical signals, and a function unit, connected to the distal end of the optical conductor, whereby signals are transmittable between the medical device and the function unit via the optical conductor.

By employing an optical conductor for carrying signals and energy, metallic conductors will not be needed for most connections between medical devices and living tissue and between different medical devices.

A refinement of the system is achieved in accordance with the invention in that the function unit comprises a first electrode surface and a second electrode surface, connected to the second conversion means so electrical signals generated by the second conversion means are delivered to the living tissue via the first electrode surface and the second electrode surface.

Hereby stimulation pulses could e.g. be delivered to a heart and accordingly replace currently known electrode systems for pacemakers. A pulse of light from e.g. a pacemaker is transmitted via the optical conductor to the second conversion means, which generates an electrical pulse to stimulate the living tissue.

Alternately, or as a complement, the function unit can comprise a first electrode surface and a second electrode surface, connected to the second conversion means, so electrical signals sensed between the first electrode surface and the second electrode surface cause the second conversion means to generate an optical signal which is sent to the first conversion means via the optical conductor.

This means that living tissue can be also sensed without the use of metallic conductors. When a combination of the two are used with a pacemaker, a heart can therefore be stimulated and sensed without the use of metallic conductors.

One alternative to emitting stimulating pulses and sensing tissue signals across one pair of electrodes is obtained in that the system is devised such, that the function unit comprises a third electrode surface and a fourth electrode surface connected to the second conversion means, so electrical signals sensed between the third electrode surface and the fourth electrode surface cause the second conversion means to generate an optical signal which is sent to the first conversion means via the optical conductor.

With two pairs of electrode surfaces, one pair for emitting stimulation pulses and one pair for sensing tissue, the system can be devised to automatically check whether an emitted stimulation pulse achieves the desired effect, i.e. whether it induces a tissue response.

In conjunction with the above-described electrode surfaces, it would be advantageous if the distance between electrode surfaces in a pair of electrodes is less than 2 mm.

With a short distance between electrode surfaces, the current needed to stimulate tissue would not need to be very high to depolarize one or several heart cells, an event sufficient to instigate a depolarization wave which stimulates all heart tissue.

An additional refinement of the device is achieved in accordance with the invention in that the function unit comprises an additional medical device, whereby information is transmittable between the medical device and the additional device via the optical conductor.

Especially in implantable systems utilizing a plurality of devices, it is advantageous if information transmission between devices is as noise-free as possible. This is achieved in accordance with the invention by the use of optical signals which are unaffected by electromagnetic fields in the surroundings, thereby making information transfer more reliable.

Additional embodiments of the system according to the invention are apparent from other sub-claims.

The medical system according to the invention will now be described in greater detail, referring to four figures in which
FIG. 1 shows an embodiment of the medical system according to the invention;
FIG. 2 shows a detailed view of a tip electrode for stimulating and sensing heart tissue;
FIG. 3 shows a contact plug for connection to a pacemaker or defibrillator;
FIG. 4 shows an alternative version for connecting an optical conductor to a pacemaker or defibrillator.

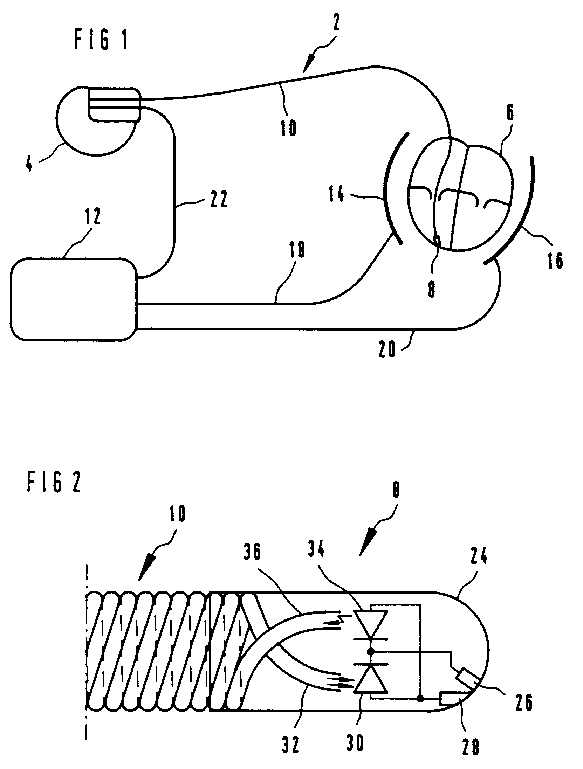

The medical system 2, shown in FIG. 1, comprises a pacemaker 4 connected to a heart 6 by an electrode element 8. The electrode element 8 and the pacemaker 4 are interconnected by a first optical line 10 for carrying signals and energy between the pacemaker 4 and the electrode element 8. Heart tissue can be sensed, or induced to trigger a heart beat, via the electrode element 8. A defibrillator 12 is also connected to the heart 6. Here, a first defibrillation electrode 14 and a second defibrillation electrode 16 are positioned so they surround the heart 6. The defibrillation electrodes 14, 16 are connected to the defibrillator 12 by a first metallic conductor 18 and a second metallic conductor 20 respectively. If the heart 6 develops fibrillation, it can be defibrillated by means of the defibrillator 12 delivering a defibrillation pulse across the first defibrillation electrode 14 and the second defibrillation electrode 16. The pacemaker 4 and the defibrillator 12 are interconnected by a second optical line 22 so they can exchange information.

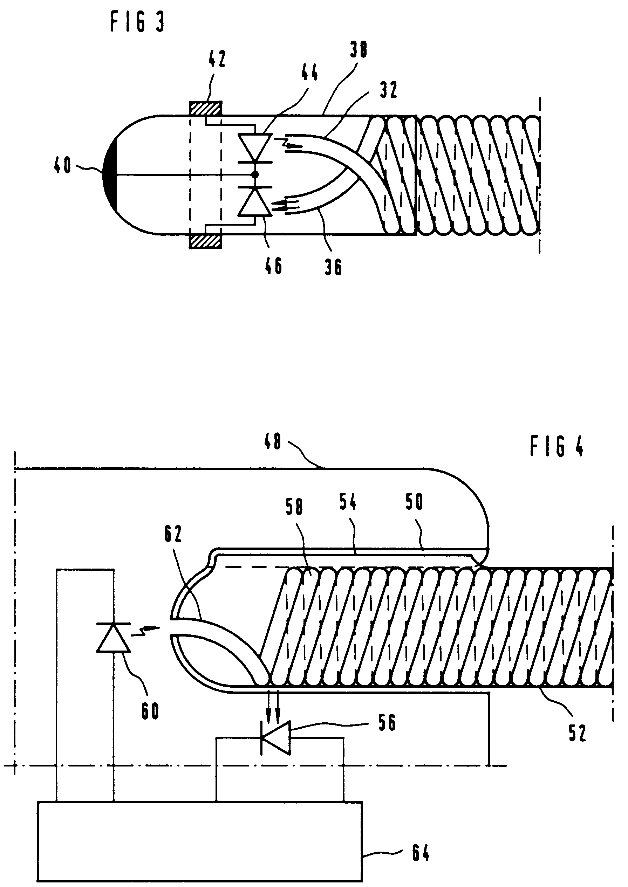

In FIG. 2 is shown the electrode element 8 in greater detail. The electrode element 8 comprises an electrode body 24, preferably made of a biocompatible polymer or some other electrically insulating optical material. A first electrode surface 26 and a second electrode surface 28 are attached to the electrode body 24. The electrode surfaces 26, 28 are positioned close to one another, e.g. at a distance of e.g. 0.5 mm. This reduces the stimulation energy needed to trigger a heart beat. A photodiode 30 is connected to the electrode surfaces 26, 28. The photodiode 30 is positioned so optical signals, carried by a first optical conductor 32 in the optical lead 10, strike it and are converted into pulses of electrical energy. Energy pulses are delivered to surrounding tissue via the first electrode surface 26 and the second electrode surface 28. A light-emitting diode 34 is also placed in the electrode body 24. The light emitting diode 34 emits light signals when electrical signals in heart tissue are picked up by the first electrode surface 26 and the second electrode surface 28. The generated optical signals are sent to a second optical conductor 36 in the optical line 10. The first optical conductor 32 and the second optical conductor 36 are helically coiled alongside each other, thereby forming the first optical line 10. When the optical conductors 32, 36 are made from a biocompatible, optical polymer, they can be helically coiled with a very narrow diameter, thereby making the first optical line 10 thinner than a corresponding metallic conductor in an insulating sheath.

FIG. 3 shows a contact plug 38 intended for detachable connection of the optical conductors 32, 36 to the pacemaker 4. The contact plug 38 has a first contact surface 40 and a second contact surface 42 in electrical contact with pacemaker electronics. A light-emitting diode 44 is connected across the first contact surface 40 and the second contact surface 42, so it can emit optical signals, carried by the first optical conductor 32, and a photodiode 46 is provided to receive optical signals carried by the second optical conductor 36 and convert same into electrical signals.

In FIG. 4 is shown an alternative way of achieving contact between the optical conductors and medical devices. A contact socket 48 on e.g. a pacemaker 4 or defibrillator 12 has a receiver receptacle 50 into which a contact plug 52 can be inserted. The contact plug 52 has a guide ridge 54 to ensure correct insertion of the contact plug 52 into the receiver receptacle 50. In the contact unit 48, there is a photodiode 56, which can receive optical signals carried by a first optical conductor 58, and a light-emitting diode 60 which can emit optical signals carried by a second optical conductor 62. The photodiode 56 and light-emitting diode 60 are connected to an electronics unit 64, e.g. pacemaker electronics, or defibrillator electronics.

## Claims

1. A medical system (2), intended to be connected to living tissue (6), comprising a medical device (4), **characterized by** at least one optical conductor (30, 32; 58, 62) with a proximal end and a distal end, for detachable connection to the medical device (4) at the proximal end, a first conversion means (44, 46; 56, 60), located at the proximal end of the optical conductor (30, 32; 58, 62) for converting optical signals into electrical signals and/or electrical signals into optical signals, a second conversion means (30, 34), located at the distal end of the optical conductor (30, 32; 58, 62) for converting optical signals into electrical signals and/or electrical signals into optical signals, and a function unit (12; 24, 26, 28), connected to the distal end of the optical conductor (30, 32; 58, 62), whereby signals are transmittable between the medical device (4) and the function unit (12; 24, 26, 28) via the optical conductor (30, 32; 58, 62).

2. A system according to claim 1, **characterized in that** the function unit (24, 26, 28) comprises a first electrode surface (26) and a second electrode surface (28), connected to the second conversion means (30), so electrical signals generated by the second conversion means (30) are delivered to the living tissue via the first electrode surface (26) and the second electrode surface (28).

3. A system according to claim 1 or 2, **characterized in that** the function unit (24, 26, 28) comprises a first electrode surface (26) and a second electrode surface (28), connected to the second conversion means (34), so electrical signals sensed between the first electrode surface (26) and the second electrode surface (28) cause the second conversion means (34) to generate an optical signal which is sent to the first conversion means (46) via the optical conductor (36).

4. A system according to claim 2, **characterized in that** the function unit comprises a third electrode surface and a fourth electrode surface connected to the second conversion means, so electrical signals sensed between the third electrode surface and the fourth electrode surface cause the second conversion means to generate an optical signal which is sent to the first conversion means via the optical conductor.

5. A system according to any of claims 2 - 4, **characterized in that** the distance between electrode surfaces in a pair of electrodes is less than 2 mm.

6. A system according to claim 1**, characterized in that** the function unit (12) consists of an additional medical device (12), information then being transmittable between the medical device (4) and the additional medical device (12) via the optical conductor (22).

7. A system according to any of the above claims, **characterized in that** the first conversion means (44, 46) comprises a light-emitting element (44), preferably a light-emitting diode or a laser diode, and the second conversion means (30, 34) comprises a current-generating element (30), preferably a photodiode.

8. A system according to any of the above claims, **characterized in that** the first conversion means (44, 46) comprises a current-generating element (46), preferably a photodiode, and the second conversion means (30, 34) comprises a light-emitting element (44), preferably a light-emitting diode or a laser diode.

9. A system according to any of the above claims, **characterized by** a contact plug (38) for detachable connection of the optical conductors (32, 36) to the medical device (4).

10. A system according to claim 9, **characterized in that** the first conversion means (44, 46) is an integral part of the contact plug (38).

11. A system according to claim 6, or either of claims 7 or 8 in combination with claim 6, **characterized by** an additional contact plug (48) arranged on the optical conductor (58, 62) for detachable connection to the additional medical device (12).

12. A system according to claim 11, **characterized in that** the second conversion means is an integral part of the additional contact plug.

13. A system according to any of the above claims, **characterized in that** the optical conductor (30, 32; 58, 62) is helically coiled.

## Patentansprüche

1. Ein zum Anschließen an lebendes Gewebe (6) vorgesehenes medizinisches System (2) mit einer medizinischen Vorrichtung (4), **gekennzeichnet** durch mindestens einen optischen Leiter (30, 32; 58, 62) mit einem proximalen Ende und einem distalen Ende zur lösbaren Verbindung mit der medizinischen Vorrichtung (4) an dem proximalen Ende, ein erstes am proximalen Ende des optischen Leiters (30, 32; 58, 62) zur Konvertierung optischer Signale in elektrische Signale und/oder elektrischer Signale in optische Signale angeordnetes Konvertierungsmittel (44, 46; 56, 60), ein zweites, am distalen Ende des optischen Leiters (30, 32; 58, 62) zur Konvertierung optischer Signale in elektrische Signal und/oder elektrischer Signale in optische Signale angeordnetes Konvertierungsmittel (30, 34) und eine mit dem distalen Ende des optischen Leiters (30, 32; 58, 62) verbundene Funktionseinheit (12; 24, 26, 28), wodurch Signale zwischen der medizinischen Vorrichtung (4) und der Funktionseinheit (12; 24, 26, 28) über den optischen Leiter (30, 32; 58, 62) übertragbar sind.

2. Ein System gemäß Anspruch 1, **dadurch gekennzeichnet,** daß die Funktionseinheit (24, 26, 28) eine erste Elektrodenoberfläche (26) und eine zweite Elektrodenoberfläche (28) aufweist, die mit dem zweiten Konvertierungsmittel (30) verbunden sind, so daß durch das zweite Konvertierungsmittel (30) erzeugte elektrische Signale über die erste Elektrodenoberfläche (26) und die zweite Elektrodenoberfläche (28) an lebendes Gewebe abgegeben werden.

3. Ein System nach Anspruch 1 oder 2, **dadurch gekennzeichnet,** daß die Funktionseinheit (24, 26, 28) eine erste Elektrodenoberfläche (26) und eine zweite Elektrodenoberfläche (28) aufweist, die mit dem zweiten Konvertierungsmittel (34) verbunden sind, so daß zwischen der ersten Elektrodenoberfläche (26) und der zweiten Elektrodenoberfläche (28) abgefühlte Signale das zweite Konvertierungsmittel (34) dazu veranlassen, ein optisches Signal zu erzeugen, das über den optischen Leiter (36) zu dem ersten Konvertierungsmittel (46) gesendet wird.

4. Ein System nach Anspruch 2, **dadurch gekennzeichnet,** daß die Funktionseinheit eine dritte Elektrodenoberfläche und eine vierte Elektrodenoberfläche aufweist, die mit dem zweiten Konvertierungsmittel verbunden sind, so daß zwischen der dritten Elektrodenoberflache und der vierten Elektrodenoberfläche abgefühlte Signale das zweite Konvertierungsmittel dazu veranlassen, ein optisches Signal zu erzeugen, das über den optischen Leiter zu dem ersten Konvertierungsmittel gesendet wird.

5. Ein System nach einem der Ansprüche 2 - 4, **dadurch gekennzeichnet,** daß der Abstand zwischen Elektrodenoberflächen in einem Paar von Elektroden kleiner als 2 mm ist.

6. Ein System nach Anspruch 1, **dadurch gekennzeichnet,** daß die Funktionseinheit (12) aus einer zusätzlichen medizinischen Vorrichtung (12) besteht, wodurch Information zwischen der medizinischen Vorrichtung (4) und der zusätzlichen medizinischen Vorrichtung (12) über den optischen Leiter (22) übertragbar ist.

7. Ein System nach einem der vorhergehenden Ansprüche, **dadurch gekennzeichnet,** daß das erste Konvertierungsmittel (44, 46) ein lichtemittierndes Element (44), vorzugsweise eine Leuchtdiode oder eine Laserdiode aufweist und das zweite Konvertierungsmittel (30, 34) ein stromerzeugendes Element (30), vorzugsweise eine Photodiode aufweist.

8. Ein System nach einem der vorhergehenden Ansprüche, **dadurch gekennzeichnet,** daß das erste Konvertierungsmittel (44, 46) ein stromerzeugendes Element (46), vorzugsweise eine Photodiode aufweist und das zweite Konvertierungsmittel (30, 34) ein lichtemittierendes Element (44), vorzugsweise eine Leuchtdiode oder eine Laserdiode aufweist.

9. Ein System nach einem der vorhergehenden Ansprüche, **gekennzeichnet** durch einen Kontaktstecker(38) zur lösbaren Verbindung des optischen Leiters (32, 36) mit der medizinischen Vorrichtung (4).

10. Ein System nach Anspruch 9, **dadurch gekennzeichnet,** daß das erste Konvertierungsmittel (44, 46) eine integraler Teil des Kontaktsteckers (38) ist.

11. Ein System nach Anspruch 6 oder entweder Anspruch 7 oder 8 in Kombination mit Anspruch 6, **gekennzeichnet** durch einen zusätzlichen, an dem optischen Leiter (58, 62) angeordneten Kontaktstecker (48) zur lösbaren Verbindung mit der zusätzlichen medizinischen Vorrichtung (12)

12. Ein System nach Anspruch 11, **dadurch gekennzeichnet,** daß das zweite Konvertierungsmittel ein integraler Teil des zusätzlichen Kontaktsteckers ist.

13. Ein System nach einem der vorhergehenden Ansprüche, **dadurch gekennzeichnet,** daß der optische Leiter (30, 32; 58, 62) wendelförmig gewickelt ist.

## Revendications

1. Système médical (2), destiné à être connecté à du tissu vivant (6), comprenant un appareil médical (4), caractérisé par au moins un conducteur optique (30, 32; 58, 62) comportant une extrémité proximale et une extrémité distale à des fins de connexion amovible à l'appareil médical (4) sur l'extrémité proximale, des premiers moyens de conversion (44, 46; 56, 60), placés à l'extrémité proximale du conducteur optique (30, 32; 58, 62) et destinés à convertir des signaux optiques en signaux électriques et/ou des signaux électriques en signaux optiques, des seconds moyens de conversion (30, 34), placés à l'extrémité distale du conducteur optique (30, 32; 58, 62) et destinés à convertir des signaux optiques en signaux électriques et/ou des signaux électriques en signaux optiques, et une unité de fonctionnement (12; 24, 26, 28), connectée à l'extrémité distale du conducteur optique (30, 32; 58, 62) et par laquelle des signaux peuvent être transmis entre l'appareil médical (4) et l'unité de fonctionnement (12; 24, 26, 28) par l'intermédiaire du conducteur optique (30, 32; 58, 62).

2. Système selon la revendication 1, caractérisé en ce que l'unité de fonctionnement (24, 26, 28) comprend une surface de première électrode (26) et une surface de seconde électrode (28), connectée aux seconds moyens de conversion (30), de sorte que des signaux électriques générés par les seconds moyens de conversion (30) sont fournis au tissu vivant par l'intermédiaire de la surface de la première électrode (26) et par la surface de la seconde électrode (28).

3. Système selon les revendications 1 ou 2, caractérisé en ce que l'unité de fonctionnement (24, 26, 28) comprend une surface de première électrode (26) et une surface de seconde électrode (28), connectées aux seconds moyens de conversion (34), de sorte que des signaux électriques détectés entre la surface de la première électrode (26) et la surface de la seconde électrode (28) font que les seconds moyens de conversion (34) engendrent un signal optique qui est envoyé aux premiers moyens de conversion (46) par l'intermédiaire du conducteur optique (36).

4. Système selon la revendication 2, caractérisé en ce que l'unité de fonctionnement comprend une surface de troisième électrode et une surface de quatrième électrode connectées aux seconds moyens de conversion, de sorte que des signaux électriques détectés entre la surface de la troisième électrode et la surface de la quatrième électrode font que les seconds moyens de conversion engendrent un signal optique qui est envoyé aux premiers moyens de conversion par l'intermédiaire du conducteur optique.

5. Système selon l'une quelconque des revendications 2 à 4, caractérisé en ce que la distance entre les surfaces d'électrode dans une paire d'électrodes est inférieure à 2 mm.

6. Système selon la revendication 1, caractérisé en ce que l'unité de fonctionnement (12) se compose d'un appareil médical supplémentaire (12), des informations pouvant alors être transmises entre l'appareil médical (4) et l'appareil médical supplémentaire (12) par l'intermédiaire du conducteur optique (22).

7. Système selon l'une quelconque des revendications ci-dessus, caractérisé en ce que les premiers moyens de conversion (44, 46) comprennent un élément électroluminescent (44), de préférence une diode électroluminescente ou une diode laser, et que les seconds moyens de conversion (30, 34) comprennent un élément générateur de courant (30), de préférence une photodiode.

8. Système selon l'une quelconque des revendications ci-dessus, caractérisé en ce que les premiers moyens de conversion (44, 46) comprennent un élément générateur de courant (46), de préférence une photodiode, et que les seconds moyens de conversion (30, 34) comprennent un élément électroluminescent (44), de préférence une diode électroluminescente ou une diode laser.

9. Système selon l'une quelconque des revendications ci-dessus, caractérisé par une fiche de contact (38) destinée à une connexion amovible des conducteurs optiques (32, 36) avec l'appareil médical.

10. Système selon la revendication 9, caractérisé en ce que les premiers moyens de conversion font partie intégrante de la fiche de contact (38).

11. Système selon la revendication 6, ou l'une des revendications 7 ou 8 en liaison avec la revendication 6, caractérisé par une fiche de contact supplémentaire (48) disposée sur le conducteur optique (58, 62) à des fins de connexion amovible sur l'appareil médical supplémentaire (12).

12. Système selon la revendication 11, caractérisé en ce que les seconds moyens de conversion font partie intégrante de la fiche de contact supplémentaire.

13. Système selon l'une quelconque des revendications ci-dessus, caractérisé en ce que le conducteur optique (30, 32; 58, 62) est enroulé en spirale.
